# EUROPEAN PATENT APPLICATION

(11) **EP 3 424 497 A1**
(43) Date of publication of application: **09.01.2019**
(21) Application number: 18175950.7
(22) Date of filing: 21.11.2012
(51) Int. Cl.: A61K 9/48, A61K 31/7004, A61P 15/08

(54) **PHARMACEUTICAL COMPOSITION COMPRISING MYO-INOSITOL AND D-CHIRO-INOSITOL**

(30) Priority: 22.11.2011 IT FI20110252
(62) Divisional of application: 12806356.7
(71) Applicant: LO. LI. Pharma S.r.l., 00156 Roma (IT)
(72) Inventor: Unfer, Vittorio, I-00155 Roma (IT)
(74) Representative: Grünecker Patent- und Rechtsanwälte PartG mbB

(57) **Abstract**

This is a description of pharmaceutical compositions comprising inositol and D-chiro-inositol in specific ratios, useful in the treatment of PCOS and for improving oocyte quality in general.

## Description

### Field of the invention

The present invention relates to the filed of pharmaceutical compositions in particular comprising mixtures of inositol and the isomers thereof.

### State of the art

Inositol, a chemical compound with formula C₆H₁₂O₆, is a carbohydrate with a different structure than common sugars. It exists in nine possible isomers, in which the most important form, widely occurring in nature, is cis-1,2,3,5-trans-4,6-cyclohexanehexol or myo-inositol.

Albeit in minimal amounts, the other existing isomers in addition to myo-inositol are scyllo-, muco-, D-chiro-, L-chiro-, neo-, allo-, epi- and cis-inositol.

Inositol play a fundamental role in the secondary messengers in cells, in the form of inositol phosphate or as phosphatidylinositol (PI) or as phosphatidylinositol phosphate (PIP).

In particular, myo-inositol takes part in important processes such as morphogenesis and cytogenesis, lipid synthesis, cell membrane formation and cell growth.

Since the work of Nestler et at in 2000, more and more scientific evidence has been accumulated in support of the physiological and therapeutic role of inositol, especially in conditions associated with polycystic ovary syndrome.

Polycystic ovary syndrome or PCOS is a complex and heterogeneous disorder affecting 6-10% of women of reproductive age and is the main cause of infertility. PCOS is primarily characterised by chronic anovulation, hyperandrogenism, an altered LH/FSH ratio (>2/3:1) and by the characteristic structure of the polycystic ovary visible on ultrasound examination. Furthermore, by inducing insulin-resistance, PCOS can increase the risk of developing glucose-intolerance, type-2 diabetes mellitus, hypertension, dyslipidemia and cardiovascular problems. Hyperinsulinaemia and hyperandrogenism are thus the two main characteristic factors of polycystic ovary syndrome, even if their cause-effect ratio continues to be subject of debate.

It has been proven that the oral administration of D-chiro-inositol or of myo-inositol has improved glucose tolerance thus reducing insulin levels in women affected by PCOS. Furthermore, it decreased the androgen level, improved ovarian function, led to reduced blood testosterone levels and improved metabolic parameters, such as arterial pressure and triglyceride levels in women affected by PCOS.

There are thus various product on the market which contain myo-inositol or D-chiro-inositol.

Nevertheless, there continues to be great medical and pharmaceutical industry demand for new formulations that prove to be even more active in lowering insulin and androgen levels in women affected by PCOS.

### Brief description of the drawings

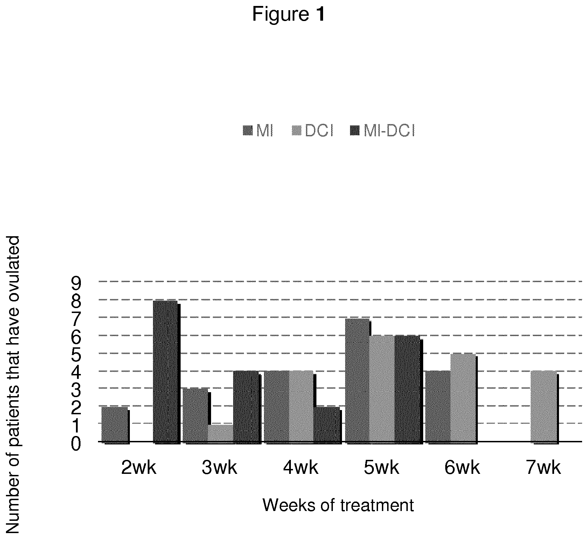

Figure 1 presents a diagram of ovulation in women affected by PCOS measured on the basis of treatment with the pharmaceutical compositions according to the invention.

### Summary of the invention

Pharmaceutical compositions are described comprising myo-inositol and D-chiro-inositol in ratios between 10:1 to 100:1 respectively (calculated by weight).

### Detailed description of the invention

The present invention allows the aforementioned needs to be met thanks to a pharmaceutical composition comprising inositol and D-chiro-inositol in specific ratios.

More specifically, the present invention relates to pharmaceutical compositions formulated for oral administration, in which the myo-inositol and the D-chiro-inositol are respectively present in a ratio between 10:1 and 100:1 (calculated by weight).

The pharmaceutical compositions are preferably formulated in a form suitable for oral administration, for example: powder, solution, tablet, soft capsule, probiotic, emulsion, micro- and nanoparticle, microemulsion, filomicelle and similar form. The compositions are prepared using the excipients commonly used in pharmacopeia in the production of said formulations, such as for example: gelatine, glycerol, cellulose, methyl cellulose, fatty acids, surfactants and emulsifiers that are processed according to known techniques.

It has been surprisingly found that in women affected by PCOS (diagnosed according to Rotterdam 2003 criteria), myo-inositol - D-chiro-inositol combination treatment is more effective than treatment with myo-inositol or D-chiro-inositol alone.

As well as proving to be effective in lowering insulin and androgen levels in women affected by PCOS, the compositions according to the invention have proved useful in treating and/or in preventing insulin resistance, hyperinsulinaemia, hyperglycaemia, hyperandrogenism, metabolic syndrome, dyslipidaemia, type-2 diabetes mellitus and cardio-cerebrovascular diseases; the compositions also find application in Medically Assisted Procreation (MAP) with a view to improving oocyte quality, optimising ovarian stimulation protocols; in particular, to prevent ovarian hyperstimulation syndrome and to improve oocyte quality.

Further beneficial effects have been observed on the classic symptoms of the menopause: irritability, hypertension, osteoporosis, dyslipidaemia, weight gain, hot flushes and aging of the skin.

In addition, the composition according to the present invention can also be used in the preparation of food bars such as: meal replacement, diet, protein bars, etc. One example of the formulation according to the invention consists of soft capsules containing:

| | |
|---|---|
| 550 mg | myo-inositol, |
| 13.8 mg | D-chiro-inositol, |
| 350 mg | soybean oil, |
| 100 mg | soy lecithin, |
| 50 mg | labrafac®, |
| 50 mg | plurol® oleique, |
| 20 mg | geleol®, |
| 0.24 mg | folic acid: |

gelatin and glycerol *quantum sufficit.*

### Preparation:

The compounds, as indicated in the soft capsule example as set out above, were mixed and weighed for the production of a batch of 10,000 capsules according to the Scherer process, which envisages:
1) the continuous and automatic preparation of two conveyor belts of get that are fed into the machine
2) formation and simultaneous filling of the capsules (the pressure of the filling liquid makes the gel expand)
3) sealing and "cutting" of the capsules by the rotary cylinders, with recovery of the gel cut-outs.
4) washing of the capsules and drying and partial dehydration thereof in infra-red tunnels or cylinders.

### Test

60 women affected by PCOS were recruited for the study and divided into three groups: 20 women were treated with 2 g of myo-inositol and 0.2 mg of folic acid twice daily, 20 women were treated with 600 mg of D-chiro-inositol and 0.2 mg of folic acid twice daily and, lastly, 20 women were treated with 550 mg of myo-inositol, 13.8 mg of D-chiro-inositol and 0.2 mg of folic acid twice daily in soft capsules.

The first parameter studied was ovulation: the results show how a greater number of patients recovers before the normal ovulation cycle if subjected to combined therapy.

In fact, as can be seen in Figure 1 hereunder, after just two weeks of combination treatment, the menstrual cycle of 8 women was re-established versus 2 women in the treatment with myo-inositol and 0 women in the treatment with D-chiro-inositol alone.

Other parameters analyses were the metabolic and hormonal pictures; the data collected is recorded in the below TABLE and relates to metabolic and hormonal values at the time of recruitment (baseline) and following treatment.

**TABLE**

| | Myo-inositol (MI) | | D-chiro-inositol (DCI) | | MI - DCI | |
|---|---|---|---|---|---|---|
| | Baseline | Treated | Baseline | Treated | Baseline | Treated |
| Testosterone ng/dl | 90,3 | 40,2 | 86,5 | 70,8 | 92,1 | 32,3 |
| DHEA (µg/dl) | 366,7 | 200,2 | 370,5 | 250,8 | 358,7 | 170,9 |
| SHGB (nmol/l) | 148,6 | 170,5 | 140,8 | 155,2 | 148,4 | 200,2 |
| LH (mIU/ml) | 15,5 | 8,1 | 13,2 | 9,8 | 17,0 | 7,8 |

The recorded data shows how the administration in a single composition of myo-inositol and D-chiro inositol, that is in line with the ratios indicated, is more effective in treating the various aspects of PCOS.

The invention is further embodied by the following items:
1. A pharmaceutical composition containing myo-inositol and D-chiro-inositol in a ratio between 10:1 to 100:1, respectively.
2. A pharmaceutical composition according to item 1 in a form suitable for oral administration.
3. A pharmaceutical composition according to item 2, wherein said form suitable for oral administration is selected from powders, solutions, tablets, soft capsules.
4. A pharmaceutical composition according to one or more of the preceding items, wherein said composition is released by probiotics.
5. A pharmaceutical composition according to one or more of the preceding items, wherein said composition is released by microemulsions, micro- or nanoparticles, or filomicelles.
6. A pharmaceutical composition according to items 1 - 6 for treating PCOS.
7. A pharmaceutical composition according to items 1 - 6 for improving the oocyte quality.
8. A pharmaceutical composition according to items 1 - 7 in the form of soft capsules having the following composition:

| | |
|---|---|
| 550 mg | myo-inositol, |
| 13.8 mg | D-chiro-inositol, |
| 350 mg | soybean oil, |
| 100 mg | soy lecithin, |
| 50 mg | labrafac, |
| 50 mg | plurol oleique, |
| 20 mg | geleol, |
| 0.24 mg | folic acid |

9. Food bars containing a composition containing myo-inositol and D-chiro-inositol in a ratio comprised between 10:1 and 100:1 respectively

## Claims

1. A pharmaceutical composition containing myo-inositol and D-chiro-inositol in a ratio between 10:1 to 100:1.

2. A pharmaceutical composition according to claim 1 in which myo-inositol and D-chiro-inositol are present in a ratio corresponding to 550:13.8.

3. A pharmaceutical composition according to claim 1 or 2 in a form suitable for oral administration.

4. A pharmaceutical composition according to claim 3, wherein said form suitable for oral administration is selected from powders, solutions, tablets, soft capsules.

5. A pharmaceutical composition according to any one of claims 1 to 3, wherein said composition is released by probiotics.

6. A pharmaceutical composition according to any one of claims 1 to 3, wherein said composition is released by microemulsions, micro- or nanoparticles, or filomicelles.

7. A pharmaceutical composition according to claims 1 - 6, for use in a method of treating polycystic ovary syndrome (PCOS).

8. A pharmaceutical composition according to claims 1 - 6, for use in a method of improving oocyte quality.

9. Food bars containing a composition containing myo-inositol and D-chiro-inositol in a ratio between 10:1 and 100:1.
